(19) Europäisches Patentamt
European Patent Office
Office européen des brevets

(11) Veröffentlichungsnummer: **0 084 819 A2**

(12) # EUROPÄISCHE PATENTANMELDUNG

(21) Anmeldenummer: **83100258.9**

(22) Anmeldetag: **14.01.83**

(51) Int. Cl.³: **A 01 N 47/36**, A 01 N 43/40, A 01 N 39/02

(30) Priorität: **26.01.82 DE 3202440**

(43) Veröffentlichungstag der Anmeldung: **03.08.83 Patentblatt 83/31**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Schmidt, Robert R., Dr., Im Waldwinkel 110, D-5060 Bergisch Gladbach 2 (DE)**

(54) **Herbizide Mittel enthaltend Methabenzthiazuron in Kombination mit bestimmten Phenoxypropionsäureestern.**

(57) Die neuen – synergistischen – Wirkstoffkombinationen, bestehend aus (1) N-(2-Benzthiazolyl)-N,N′-dimethylharnstoff (Methabenzthiazuron) der Formel I

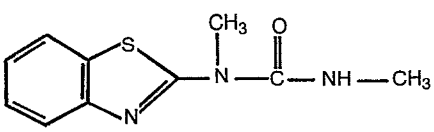

(I)

und (2) mindestens einem substituierten Phenoxypropionsäureester der allgemeinen Formel II

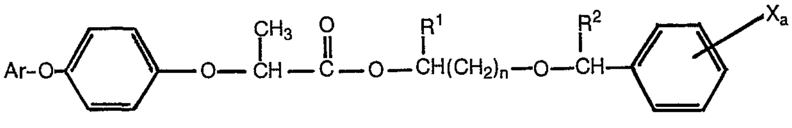

(II)

(worin die Symbole $R^1$, $R^2$, Ar, X, a und n die in der Beschreibung angegebene Bedeutung haben), können zur selektiven Unkrautbekämpfung in verschiedenen Kulturen, vorzugsweise in Getreidekulturen, verwendet werden, wobei auch sonst schwer bekämpfbare Unkräuter bzw. Ungräser wie Flughafer und hirseartige Ungräser sicher miterfaßt werden.

EP 0 084 819 A2

0084819

BAYER AKTIENGESELLSCHAFT 5090 Leverkusen, Bayerwerk

Zentralbereich
Patente, Marken und Lizenzen Bi/m-c

III

# Herbizide Mittel enthaltend Methabenzthiazuron in Kombination mit bestimmten Phenoxypropionsäureestern

Die Erfindung betrifft neue herbizide, synergistische Wirkstoffkombinationen, die aus Methabenzthiazuron einerseits und bestimmten Phenoxypropionsäureestern andererseits bestehen und mit Vorteil zur selektiven Unkrautbekämpfung in verschiedenen Kulturen, vorzugsweise in Getreidekulturen, verwendet werden können.

Es ist bereits bekannt geworden, daß N-(2-Benzthiazolyl)-N,N'-dimethylharnstoff herbizide Eigenschaften besitzt (vgl. US-PS 2 756 135). Ferner ist es bekannt, daß der N-(2-Benzthiazolyl)-N,N'-dimethylharnstoff (common name: Methabenzthiazuron) als Selektiv-Herbizid in Gerste und Weizen verwendet werden kann (vgl. z.B. R. Wegler, Chemie der Pflanzenschutz- und Schädlingsbekämpfungsmittel, Band 5 "Herbizide", Springer-Verlag 1977, S. 145-146; DE-PS 15 42 789). Es hat sich jedoch gezeigt, daß Methabenzthiazuron gegen Flughafer und gegen hirseartige Ungräser nur schwach wirksam ist.

Es wurde nun überraschend gefunden, daß die neuen Wirkstoffkombinationen bestehend aus

(1) N-(2-Benzthiazolyl)-N,N'-dimethylharnstoff der Formel I

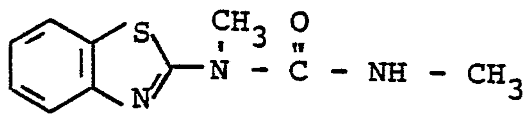

(I)

und

(2) mindestens einem substituierten Phenoxypropionsäureester der allgemeinen Formel II

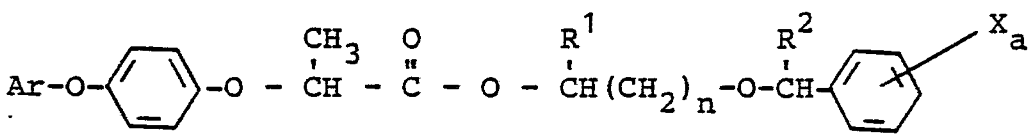

(II)

worin

$R^1$ für Wasserstoff oder Methyl steht,

$R^2$ für Wasserstoff oder Methyl steht,

n für 1 oder 2 steht,

X für Wasserstoff, Methyl, Methoxy, Halogen und/oder Nitro steht,

a für 1 oder 2 steht,

Ar für den Rest 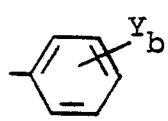 oder 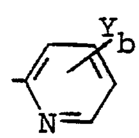 steht,

Y für $C_{1-4}$-Alkyl, Halogen, Trifluormethyl, Nitro und/oder Cyano steht und

b für 1, 2 oder 3 steht,

eine besonders hohe herbizide Wirksamkeit aufweisen.

Besonders bevorzugte Wirkstoffe gemäß Formel (II) sind die folgenden Verbindungen:

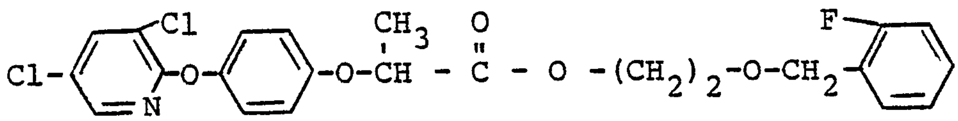

(II-1)

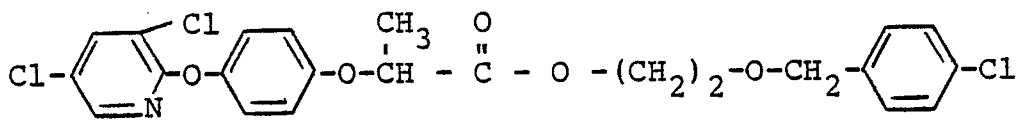

(II-2)

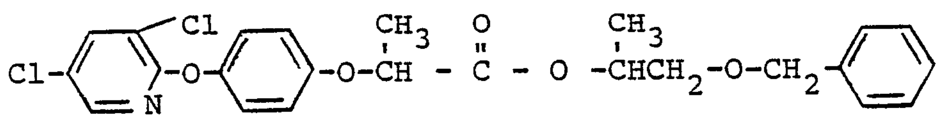

(II-3)

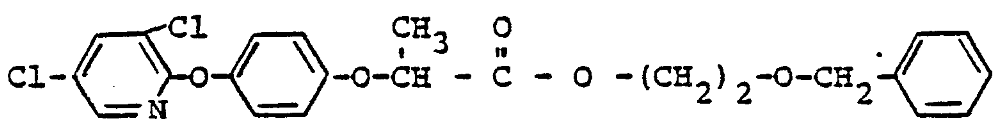

(II-31)

Überraschenderweise ist die herbizide Wirksamkeit der erfindungsgemäßen Wirkstoffkombinationen wesentlich höher als die Summe der Wirkungen der einzelnen Wirkstoffe. Es liegt somit ein nicht vorhersehbarer echter synergistischer Effekt vor und nicht nur eine Wirkungsergänzung. Die neuen Wirkstoffkombinationen sind ebenso wie Methabenzthiazuron (I) in bestimmten Getreidearten, wie Weizen, sehr gut verträglich, wobei die neuen Wirkstoffkombinationen auch die "Problemunkräuter" - Flughafer und hirseartige Ungräser - hervorragend bekämpfen. Die neuen Wirkstoffkombinationen stellen somit eine wertvolle Bereicherung der Selektivherbizide, insbesondere der Getreideherbizide, dar.

Als Unkräuter, die im allgemeinen als Verunreinigung in Getreidekulturen auftreten und durch die erfindungsgemäßen Wirkstoffkombinationen sicher bekämpft werden können, seien beispielsweise genannt:

dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea; sowie

monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen weisen bei sehr guter Verträglichkeit gegenüber Getreidekulturen eine hervorragende Wirkung gegen Unkräuter und Ungräser auf, insbesondere gegen hirseartige Ungräser. Ihr Einsatz als selektive Getreideherbizide ist daher besonders bevorzugt.

Der synergistische Effekt der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 - 20 Gewichtsteile, vorzugsweise 0,01 - 10 Gewichtsteile Wirkstoff aus der Wirkstoffgruppe (II).

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder

chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: Z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige

körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, zum Beispiel Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Getreide-Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombination können in einem gewissen Bereich variiert werden; sie hängen u.a. vom Wetter und von den Bodenfaktoren ab. Im allgemeinen liegen die Aufwandmengen zwischen 0,01 und 10 kg Wirkstoffkombination pro ha, vorzugsweise zwischen 0,05 und 5 kg/ha.

Die erfindungsgemäßen Wirkstoffkombinationen können sowohl vor als auch nach dem Auflaufen der Pflanzen appliziert werden. Die Anwendung wird vorzugsweise nach dem Auflaufen der Pflanzen, also im post-emergence-Verfahren, vorgenommen.

Die gute herbizide Wirkung der neuen Wirkstoffkombinationen geht aus den nachfolgenden Beispielen hervor. Während die einzelnen Wirkstoffe in der herbiziden Wirkung Schwächen aufweisen, zeigen die Kombinationen eine sehr breite Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die der einzeln applizierten Wirkstoffe.

Die zu erwartende Wirkung für eine gegebene Kombination zweier Herbizide kann wie folgt berechnet werden (vgl. COLBY, S.R., "Calculating synergistic and antagonistic responses of herbicide combinations", Weeds 15, Seiten 20-22, 1967):

Wenn X = % Schädigung durch Herbizid A bei p kg/ha Aufwandmenge und

Y = % Schädigung durch Herbizid B bei q kg/ha Aufwandmenge und

E = die erwartete Schädigung der Herbizide A und B, bei p und q kg/ha Aufwandmenge,

dann ist $E = X + Y - \frac{X \cdot Y}{100}$ .

Ist die tatsächliche Schädigung größer als berechnet, so ist die Kombination in ihrer Wirkung überadditiv, d.h. sie zeigt einen synergistischen Effekt.

Aus den nachfolgenden Beispielen geht hervor, daß die gefundene herbizide Wirkung der erfindungsgemäßen Wirkstoffkombinationen bei den Unkräutern größer ist als die berechnete, d.h., daß die neuen Wirkstoffkombinationen synergistisch wirken.

Die erfindungsgemäßen Wirkstoffkombinationen zeigen zum Teil auch eine Nebenwirkung gegen Bodeninsekten sowie eine systemische Wirkung gegen Pyricularia oryzae an Reis, welche auf einer entsprechenden Wirkung der Wirkstoffkomponente (II) beruhen dürfte.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die in der Tabelle angegebenen Wirkstoffmengen pro Flächeneinheit ausgebracht werden. Die Konzentration der Spritzbrühe wird so gewählt, daß in 2000 l Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle A hervor:

## Tabelle A

Post-emergence-Test / Gewächshaus

| Wirkstoff bzw. Wirkstoffkombination ** | Aufwandmenge kg/ha | Schädigung bzw. Wirkung in % | | | | | | | | | |
|---|---|---|---|---|---|---|---|---|---|---|---|
| | | Weizen | | Avena fatua | | Digitaria | | Galium | | Echinochloa | |
| | | gef.* | ber.* | gef.* | ber.* | gef.* | ber.* | gef.* | ber.* | gef.* | ber.* |
| (I) - bekannt - | 1,4 | 0 | | 0 | | 70 | | 50 | | 40 | |
| (II-1) | 0,07 | 0 | | 30 | | 20 | | 0 | | 30 | |
| (II-2) | 0,07 | 0 | | 30 | | 20 | | 0 | | 30 | |
| (II-3) - bekannt - | 0,07 | 0 | | 0 | | 30 | | 0 | | 50 | |
| (I)+(II-1) | 1,4 + 0,07 | 10 | 0 | 50 | 30 | 99 | 76 | 90 | 50 | 90 | 58 |
| (I)+(II-2) | 1,4 + 0,07 | 10 | 0 | 60 | 30 | 99 | 76 | 90 | 50 | 99 | 58 |
| (I)+(II-3) -erfindungsgemäß- | 1,4 + 0,07 | 0 | 0 | 60 | 0 | 99 | 79 | 95 | 50 | 99 | 70 |

* gef. = gefundene Schädigung (in Prozent)

* ber. = nach der COLBY-Formel berechnete Schädigung (in Prozent)

** Wirkstoff (I) = Methabenzthiazuron; die Strukturformeln der übrigen Wirkstoffe sind mit gleicher Nummer unter den Herstellungsbeispielen angegeben.

Der Wirkstoff der Formel (I) ist bekannt (US-PS 2 756 135). Die Wirkstoffe der Formel (II) sind noch nicht druckschriftlich beschrieben worden; sie sind Gegenstand der JP-Patentanmeldung Nr. Sho 56-97486 vom 25.6.1981 sowie der JP-Patentanmeldung Nr. Sho 56-144 778 vom 16.9.1981. Die Wirkstoffe der allgemeinen Formel II

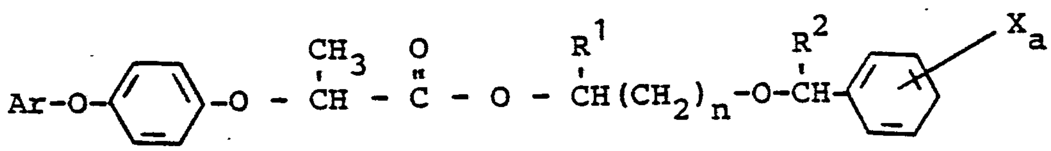

(II)

worin

$R^1$ für Wasserstoff oder Methyl steht,

$R^2$ für Wasserstoff oder Methyl steht,

n für 1 oder 2 steht,

X für Wasserstoff, Methyl, Methoxy, Halogen und/oder Nitro steht,

a für 1 oder 2 steht,

Ar für den Rest 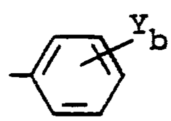 oder 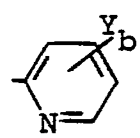 steht,

Y für $C_{1-4}$-Alkyl, Halogen, Trifluormethyl, Nitro und/oder Cyano steht und

b für 1, 2 oder 3 steht,

können hergestellt werden, indem man entweder

(a) eine Verbindung der allgemeinen Formel III

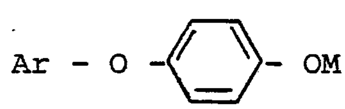

(III)

worin

M für Wasserstoff oder ein Alkalimetall-Atom steht und
Ar die oben angegebene Bedeutung hat,

mit einer Verbindung der allgemeinen Formel IV

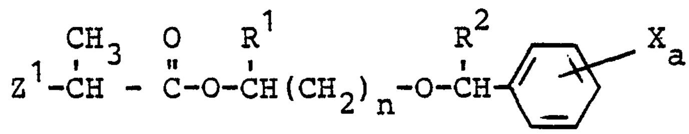
(IV)

worin
$Z^1$ für Halogen steht und
$R^1$, $R^2$, X, n und a die oben angegebene Bedeutung haben,

umsetzt oder indem man

(b) eine Verbindung der allgemeinen Formel V

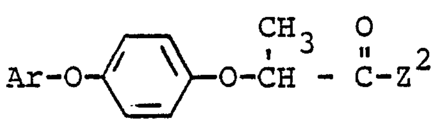
(V)

worin
$Z^2$ für Hydroxy oder Halogen steht und
Ar die oben angegebene Bedeutung hat,

mit einer Verbindung der allgemeinen Formel VI

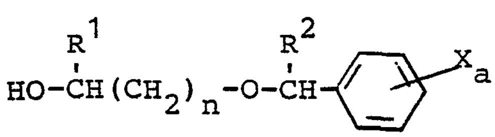
(VI)

worin

$R^1$, $R^2$, X, n und a die oben angegebene Bedeutung haben,

umsetzt.

Verwendet man nach Verfahrensweg (a) beispielsweise 4-(3,5-Dichlor-2-pyridyloxy)-phenol-Natriumsalz und 2-Brompropionsäure-/2-(2-fluorbenzyloxy)/-ethylester als Ausgangsstoffe, so läßt sich der Reaktionsablauf durch das folgende Formelschema wiedergeben:

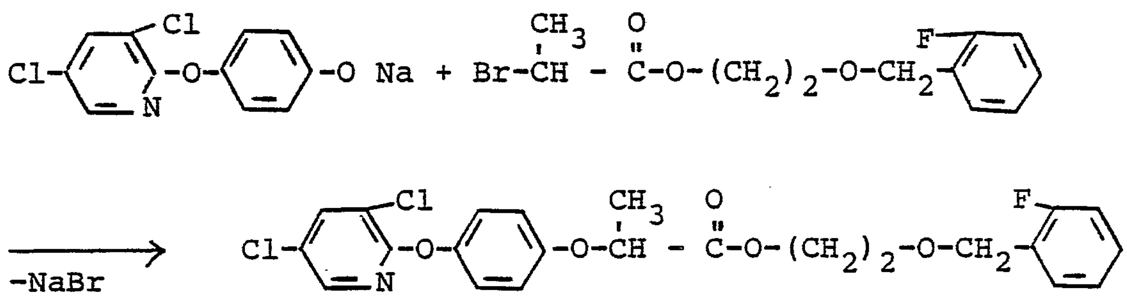


Als bevorzugte Beispiele für Ausgangsstoffe der Formel (III) sind zu nennen:

4-(4-Trifluormethylphenoxy)-phenol,
4-(2-Trifluormethylphenoxy)-phenol,
4-(4-Fluorphenoxy)-phenol,
4-(2,4-Dichlorphenoxy)-phenol,
4-(2-Chlor-4-nitrophenoxy)-phenol,
4-(2-Trifluormethyl-4-chlorphenoxy)-phenol,
4-(4-Trifluormethyl-2-chlorphenoxy)-phenol,
4-(3,5-Dichlor-2-pyridyloxy)-phenol,
4-(5-Nitro-2-pyridyloxy)-phenol,
4-(4-Nitrophenoxy)-phenol

0084819

4-(4-Brom-2-chlorphenoxy)-phenol,
4-(4-Trifluormethyl-2-nitrophenoxy)-phenol,
4-(2,6-Dichlor-4-trifluormethylphenoxy)-phenol,
4-(2-Cyano-4-trifluormethylphenoxy)-phenol,
4-(2-Chlor-4-cyanophenoxy)-phenol,
4-(3-Chlor-5-nitro-2-pyridyloxy)-phenol,
4-(5-Trifluormethyl-2-pyridyloxy)-phenol,
4-(3-Chlor-5-trifluormethyl-2-pyridyloxy)-phenol,
4-(5-Brom-3-chlor-2-pyridyloxy)-phenol und
4-(4-Chlor-2-methylphenoxy)-phenol;

es können auch die Alkalimetallsalze, vorzugsweise die Natrium- und Kaliumsalze, der vorgenannten Phenole eingesetzt werden.

Als bevorzugte Ausgangsstoffe der Formel (IV) sind zu nennen:

2-Benzyloxyethyl-2-chlor-(oder -brom)-propionat,
3-Benzyloxypropyl-2-chlor-(oder -brom)-propionat,
1-Methyl-2-benzyloxyethyl-2-chlor-(oder -brom)-propionat,
2-α-Methylbenzyloxyethyl-2-chlor-(oder -brom)-propionat,
2-(2-Fluorbenzyloxy)-ethyl-2-chlor-(oder -brom)-propionat,
2-(2-Chlorbenzyloxy)-ethyl-2-chlor-(oder -brom)-propionat,
2-(4-Chlorbenzyloxy)-ethyl-2-chlor-(oder -brom)-propionat,
2-(2,4-Dichlorbenzyloxy)-ethyl-2-chlor-(oder -brom)-propionat,
2-(3,4-Dichlorbenzyloxy)-ethyl-2-chlor-(oder -brom)-propionat,
2-(2,6-Dichlorbenzyloxy)-ethyl-2-chlor-(oder -brom)-propionat,
2-(4-Fluorbenzyloxy)-ethyl-2-chlor-(oder -brom)-propionat,

2-(2-Methylbenzyloxy)-ethyl-2-chlor-(oder -brom)-propionat,
2-(3-Nitrobenzyloxy)-ethyl-2-chlor-(oder -brom)-propionat,
2-(4-Methoxybenzyloxy)-ethyl-2-chlor-(oder -brom)-propionat,
2-(4-Brombenzyloxy)-ethyl-2-chlor-(oder -brom)-propionat,
1-Methyl-2-α-methylbenzyloxyethyl-2-chlor-(oder -brom)-propionat,
2-(3-Chlorbenzyloxy)-ethyl-2-chlor-(oder -brom)-propionat,
2-(2-Brombenzyloxy)-ethyl-2-chlor-(oder -brom)-propionat,
2-(3-Fluorbenzyloxy)-ethyl-2-chlor-(oder -brom)-propionat,
1-Methyl-2-(2-fluorbenzyloxy)-ethyl-2-chlor-(oder -brom)-propionat,
3-(2-Fluorbenzyloxy)-propyl-2-chlor-(oder -brom)-propionat,
2-(2-Nitrobenzyloxy)-ethyl-2-chlor-(oder -brom)-propionat,
2-(4-Nitrobenzyloxy)-ethyl-2-chlor-(oder -brom)-propionat,
2-(4-Methylbenzyloxy)-ethyl-2-chlor-(oder -brom)-propionat,
2-(2-Methoxybenzyloxy)-ethyl-2-chlor-(oder -brom)-propionat,
und
3-(4-Fluorbenzyloxy)-propyl-2-chlor-(oder -brom)-propionat.

Verwendet man nach Verfahrensweg (b) beispielsweise 2-/4-(5-Trifluormethyl-2-pyridyloxy)-phenoxy/-propionylchlorid und 1-(4-Chlorbenzyloxy)-2-propanol als Ausgangsstoffe, so läßt sich der Reaktionsablauf durch das fol-

gende Formelschema wiedergeben:

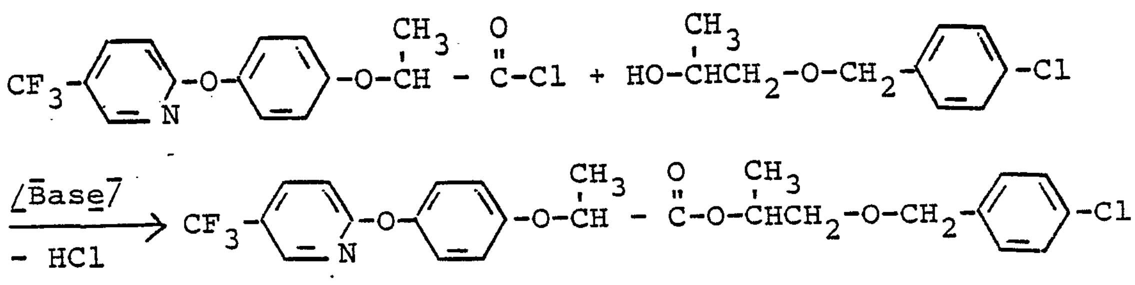


Als bevorzugte Beispiele für Ausgangsstoffe der Formel (V) sind zu nennen:

2-[4-(4-Trifluormethylphenoxy)-phenoxy]-propionyl-chlorid,
2-[4-(2-Trifluormethylphenoxy)-phenoxy]-propionyl-chlorid,
2-[4-(4-Fluorphenoxy)-phenoxy]-propionyl-chlorid,
2-[4-(2,4-Dichlorphenoxy)-phenoxy]propionyl-chlorid,
2-[4-(2-Chlor-4-nitrophenoxy)-phenoxy]-propionyl-chlorid,
2-[4-(2-Trifluormethyl-4-chlorphenoxy)-phenoxy]-propionyl-chlorid,
2-[4-(3,5-Dichlor-2-pyridyloxy)-phenoxy]-propionyl-chlorid,
2-[4-(4-Trifluormethyl-2-chlorphenoxy)-phenoxy]-propionyl-chlorid,
2-[4-(5-Nitro-2-pyridyloxy)-phenoxy]-propionyl-chlorid,
2-[4-(4-Nitrophenoxy)-phenoxy]-propionyl-chlorid,
2-[4-(4-Brom-2-chlorphenoxy)-phenoxy]-propionyl-chlorid,
2-[4-(4-Trifluormethyl-2-nitrophenoxy)-phenoxy]-propionyl-chlorid,
2-[4-(2,6-Dichlor-4-trifluormethylphenoxy)-phenoxy]-propionyl-chlorid,

2-[4-(2-Cyano-4-trifluormethylphenoxy)-phenoxy]-propionyl-chlorid,
2-[4-(2-Chlor-4-cyanophenoxy)-phenoxy]-propionyl-chlorid,
2-[4-(3-Chlor-5-nitro-2-pyridyloxy)-phenoxy]-propionyl-chlorid,
2-[4-(5-Trifluormethyl-2-pyridyloxy)-phenoxy]-propionyl-chlorid,
2-[4-(3-Chlor-5-trifluormethyl-2-pyridyloxy)-phenoxy]-propionyl-chlorid,
2-[4-(5-Brom-3-chlor-2-pyridyloxy)-phenoxy]-propionyl-chlorid und
2-[4-(4-Chlor-2-methylphenoxy)-phenoxy]-propionyl-chlorid;

anstelle der Säurechloride können auch die entsprechenden Säurebromide oder freien Propionsäuren eingesetzt werden.

Als bevorzugte Beispiele für Ausgangsstoffe der Formel (VI) sind zu nennen:

2-Benzyloxyethanol,
3-Benzyloxypropanol,
1-Benzyloxy-2-propanol,
2-α-Methylbenzyloxyethanol,
2-(2-Fluorbenzyloxy)-ethanol,
2-(4-Fluorbenzyloxy)-ethanol,
2-(2-Chlorbenzyloxy)-ethanol,
2-(4-Chlorbenzyloxy)-ethanol,
2-(2,4-Dichlorbenzyloxy)-ethanol,

2-(3,4-Dichlorbenzyloxy)-ethanol,
2-(2,6-Dichlorbenzyloxy)-ethanol,
2-(2-Methylbenzyloxy)-ethanol,
2-(3-Nitrobenzyloxy)-ethanol,
2-(4-Methoxybenzyloxy)-ethanol,
2-(4-Brombenzyloxy)-ethanol,
1-(α-Methylbenzyloxy)-2-propanol,
2-(3-Chlorbenzyloxy)-ethanol,
2-(2-Brombenzyloxy)-ethanol,
2-(3-Fluorbenzyloxy)-ethanol,
1-(2-Fluorbenzyloxy)-2-propanol,
3-(2-Fluorbenzyloxy)-propanol,
2-(2-Nitrobenzyloxy)-ethanol,
2-(4-Nitrobenzyloxy)-ethanol,
2-(4-Methylbenzyloxy)-ethanol,
2-(2-Methoxybenzyloxy)-ethanol und
3-(4-Fluorbenzyloxy)-propanol.

Beide Verfahren werden bevorzugt in Gegenwart eines Verdünnungsmittels durchgeführt. Geeignete Verdünnungsmittel sind Wasser sowie inerte organische Lösungsmittel, z.B. gegebenenfalls chlorierte Kohlenwasserstoffe wie Benzol oder Chloroform, Ether wie Dioxan und Tetrahydrofuran, Nitrile wie Acetonitril, Sulfone und Sulfoxide wie Dimethylsulfoxid bzw. Sulfolan oder tertiäre Basen wie Pyridin.

Beide Verfahren werden bevorzugt in Gegenwart eines Säurebindemittels durchgeführt. Hierfür kommen alle üblicherweise verwendeten Säurebinder in Frage, z.B.

Alkalihydroxide, -carbonate, -bicarbonate und -alkoholate, oder tertiäre Amine wie Triethylamin, Diethylanilin oder Pyridin.

Die Reaktionstemperaturen können bei beiden Verfahren in einem größeren Bereich variiert werden. Im allgemeinen arbeitet man bei Temperaturen zwischen -20°C und dem Siedepunkt des jeweiligen Reaktionsgemisches, vorzugsweise zwischen 0°C und 100°C.

Bei der Durchführung der beiden Verfahren werden die Ausgangsstoffe im allgemeinen in etwa äquimolaren Mengen eingesetzt. Säurebindemittel werden in stöchiometrischen oder bis zu dreifach überstöchiometrischen Mengen eingesetzt.

Die Aufarbeitung und Isolierung der Reaktionsprodukte (II) erfolgt in üblicher Weise. Die folgenden Herstellungsbeispiele dienen zur weiteren Erläuterung der beschriebenen Herstellungsverfahren.

Herstellungsbeispiele

Beispiel 1 - nach Verfahren (a)

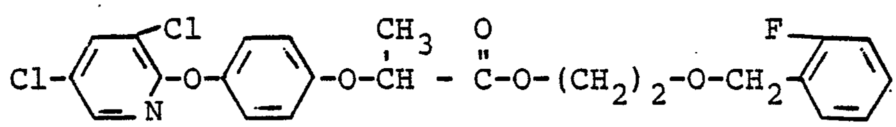

(II-1)

Eine Mischung von 30.6 g 4-(3,5-Dichlor-2-pyridyloxy)-phenol-Natriumsalz und 36 g 2-Brompropionsäure-/2-(2-fluorbenzyloxy)7-ethylester (IV-1) in 100 ml Dimethylformamid wurde für 3 Stunden unter Rühren auf 70-80°C erwärmt. Nach Abkühlung auf Raumtemperatur wurde das Reaktionsgemisch in 300 ml Wasser gegossen, dann wurde mit Ether extrahiert; die etherische Phase wurde zunächst mit 1 %-Natronlauge, dann mit Wasser gewaschen und getrocknet. Nach Abdestillieren des Ethers erhält man als Rückstand 38 g des gewünschten 2-/4-(3,5-Dichlor-2-pyridyloxy)-phenoxy7-propionsäure-/2-(2-fluorbenzyloxy)7-ethylesters (II-1); Schmelzpunkt 54-57°C.

Beispiel 2 - nach Verfahren (a)

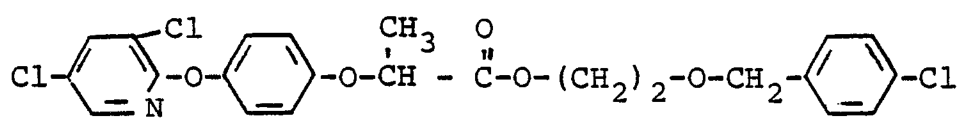

(II-2)

Analog zur Beispiel 1 wurde durch Umsetzung von 4-(3,5-Dichlor-2-pyridyloxy)-phenol-Natriumsalz mit 2-Brompro-

pionsäure-[2-(4-chlorbenzyloxy)]-ethylester (IV-6) die Verbindung 2-[4-(3,5-Dichlor-2-pyridyloxy)-phenoxy]-propionsäure-[2-(4-chlorbenzyloxy)]-ethylester (II-2) hergestellt; $n_D^{20}$: 1,5803

Beispiel 3 - nach Verfahren (b)

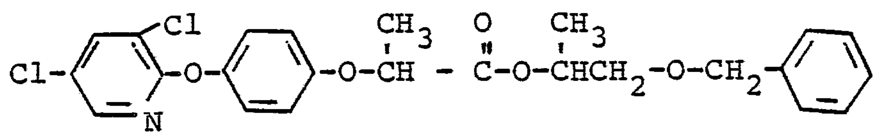
(II-3)

16,6 g 1-Benzyloxy-2-propanol und 10,6 g Triethylamin wurden in 200 ml Toluol gelöst, dann auf 0°C abgekühlt. Zu dieser Lösung wurde bei 0-5°C unter Rühren eine Lösung von 34,7 g 2-[4-(3,5-Dichlor-2-pyridyloxy)-phenoxy]-propionylchlorid in 80 ml Toluol zugetropft. Das Reaktionsgemisch wurde anschließend langsam auf 40-50°C erwärmt und zur Vervollständigung der Umsetzung eine weitere Stunde gerührt. Nach der Aufarbeitung (analog zu Beispiel 1) wurden 43.4 g 2-[4-(3,5-Dichlor-2-pyridyloxy)-phenoxy]-propionsäure-(2-benzyloxy-1-methyl)-ethylester (II-3) in Form eines viskosen Öls erhalten; $n_D^{20}$: 1,5710.

In analoger Weise können auch die in der folgenden Tabelle 1 aufgeführten Verbindungen der allgemeinen Formel (II) hergestellt werden:

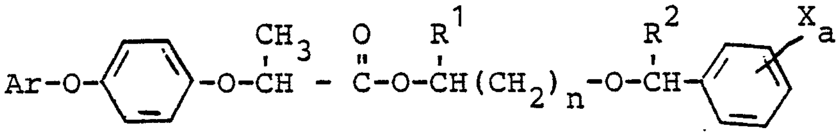 (II)

Tabelle 1

| Verbindung Nr. | Ar | $R^1$ | $R^2$ | n | $X_a$ | Physical. Konstante |
|---|---|---|---|---|---|---|
| II-4 | 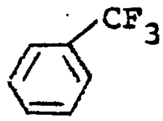 | H | H | 1 | H | $n_D^{20}$ 1.5322 |
| II-5 | 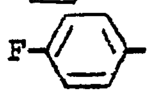 | H | H | 1 | H | $n_D^{20}$ 1.5518 |
| II-6 | 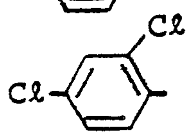 | H | H | 1 | H | $n_D^{20}$ 1.5696 |
| II-7 | 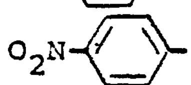 | H | H | 1 | H | $n_D^{20}$ 1.5805 |
| II-8 | 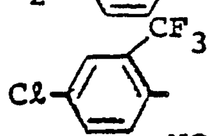 | H | H | 1 | H | $n_D^{20}$ 1.5397 |
| II-9 | 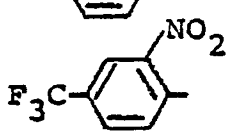 | H | H | 1 | H | $n_D^{20}$ 1.5450 |
| II-10 | 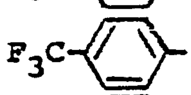 | H | H | 2 | H | $n_D^{20}$ 1.5268 |
| II-11 | 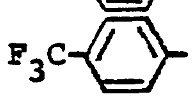 | $-CH_3$ | H | 1 | H | $n_D^{20}$ 1.5260 |
| II-12 | 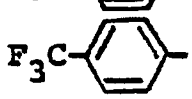 | H | $-CH_3$ | 1 | H | $n_D^{20}$ 1.5266 |
| II-13 | 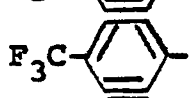 | H | H | 1 | 2-F | $n_D^{20}$ 1.5235 |
| II-14 | 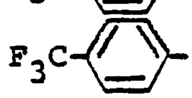 | H | H | 1 | 4-F | $n_D^{20}$ 1.5214 |

Tabelle 1 (Fortsetzung)

| Verbindung Nr. | Ar | $R^1$ | $R^2$ | n | $X_a$ | Physical. Konstante |
|---|---|---|---|---|---|---|
| II-15 | 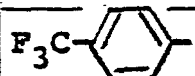 | H | H | 1 | 2-Cl | $n_D^{20}$ 1.5360 |
| II-16 | 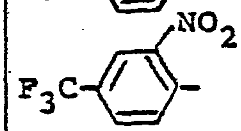 | H | H | 1 | 2-Cl | $n_D^{20}$ 1.5550 |
| II-17 | 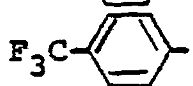 | H | H | 1 | 4-Cl | $n_D^{20}$ 1.5360 |
| II-18 | 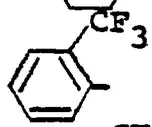 | H | H | 1 | 4-Cl | $n_D^{20}$ 1.5380 |
| II-19 | 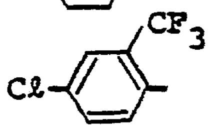 | H | H | 1 | 4-Cl | $n_D^{20}$ 1.5450 |
| II-20 | 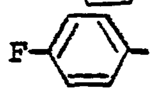 | H | H | 1 | 4-Cl | $n_D^{20}$ 1.5559 |
| II-21 | 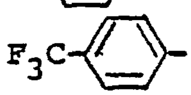 | H | H | 1 | 2,4-$Cl_2$ | $n_D^{20}$ 1.5422 |
| II-22 | 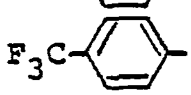 | H | H | 1 | 3,4-$Cl_2$ | $n_D^{20}$ 1.5428 |
| II-23 | 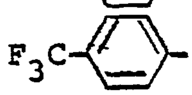 | H | H | 1 | 2-$CH_3$ | $n_D^{20}$ 1.5305 |
| II-24 | 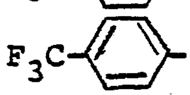 | H | H | 1 | 3-$NO_2$ | $n_D^{20}$ 1.5436 |
| II-25 | 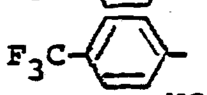 | H | H | 1 | 4-$OCH_3$ | $n_D^{20}$ 1.5343 |
| II-26 | 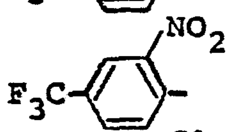 | H | H | 2 | H | $n_D^{20}$ 1.5440 |
| II-27 | 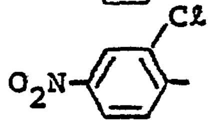 | H | H | 1 | 4-Cl | $n_D^{20}$ 1.5875 |
| II-28 | 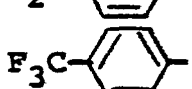 | H | H | 1 | 4-Br | $n_D^{20}$ 1.5450 |
| II-29 | 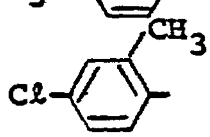 | H | H | 1 | H | $n_D^{20}$ 1.5650 |

Tabelle 1 (Fortsetzung)

| Verbindung Nr. | Ar | $R^1$ | $R^2$ | n | $X_a$ | Physical. Konstante |
|---|---|---|---|---|---|---|
| II-30 | 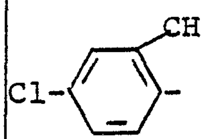 | H | H | 1 | 2-Cl | $n_D^{20}$ 1.5718 |
| II-31 | 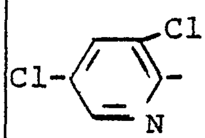 | H | H | 1 | H | Fp.59-61°C |
| II-32 | 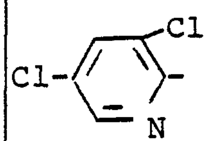 | H | $-CH_3$ | 1 | H | $n_D^{20}$ 1.5715 |
| II-33 | 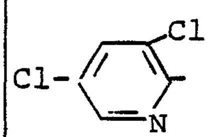 | H | H | 2 | H | $n_D^{20}$ 1.5721 |
| II-34 | 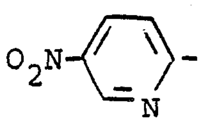 | H | H | 1 | H | Öl |
| II-35 | 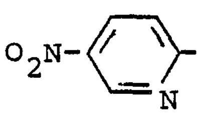 | H | H | 1 | $3-NO_2$ | Öl |
| II-36 | 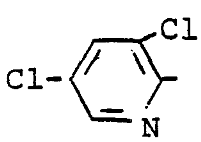 | H | H | 1 | 2-Cl | Fp. 64-66°C |
| II-37 | 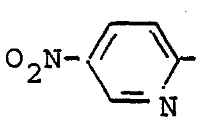 | H | H | 1 | 4-Cl | Öl |
| II-38 | 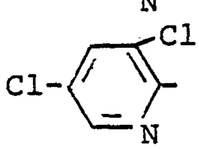 | H | H | 1 | $3-NO_2$ | $n_D^{20}$ 1.5845 |

Tabelle 1 (Fortsetzung)

| Verbindung Nr. | Ar | $R^1$ | $R^2$ | n | $X_a$ | Physical. Konstante |
|---|---|---|---|---|---|---|
| II-39 | 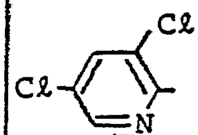 | H | H | 1 | $2-CH_3$ | Fp. 83-85°C |
| II-40 | 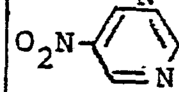 | H | H | 1 | $2,4-Cl_2$ | Öl |
| II-41 | 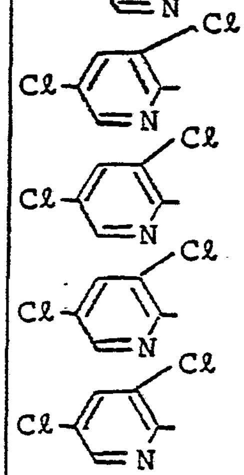 | H | H | 1 | $3,4-Cl_2$ | $n_D^{20}$ 1.5900 |
| II-42 | | H | H | 1 | $2,6-Cl_2$ | $n_D^{20}$ 1.5915 |
| II-43 | | H | H | 1 | $4-OCH_3$ | $n_D^{20}$ 1.5735 |
| II-44 | | H | H | 1 | 4-Br | $n_D^{20}$ 1.5870 |
| II-45 | 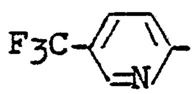 | H | $CH_3$ | 1 | H | $n_D^{20}$ 1,5254 |
| II-46 | 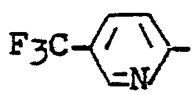 | H | H | 1 | $2-CH_3$ | $n_D^{20}$ 1,5273 |
| II-47 | 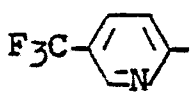 | H | H | 1 | 2-Cl | $n_D^{20}$ 1,5349 |
| II-48 | 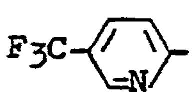 | H | H | 1 | 4-Br | $n_D^{20}$ 1,5440 |
| II-49 | 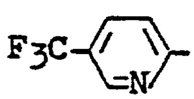 | H | H | 1 | $3-NO_2$ | $n_D^{20}$ 1,5428 |
| II-50 | 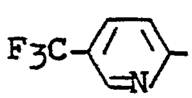 | H | H | 1 | $2,4-Cl_2$ | $n_D^{20}$ 1,5424 |

Tabelle 1 (Fortsetzung)

| Verbindung Nr. | Ar | $R^1$ | $R^2$ | n | Xa | Physikalische Konstante |
|---|---|---|---|---|---|---|
| II-51 | 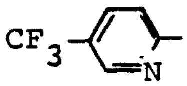 | H | H | 1 | H | Fp. 44-47°C |
| II-52 | 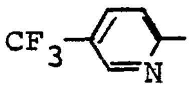 | H | H | 1 | 4-Cl | $n_D^{20}$ 1,5350 |
| II-53 | 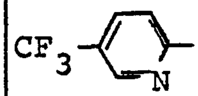 | $CH_3$ | H | 1 | H | $n_D^{20}$ 1,5224 |
| II-54 | 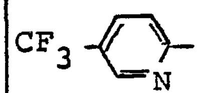 | H | H | 2 | H | $n_D^{20}$ 1,5245 |
| II-55 | 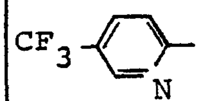 | H | H | 1 | 2-F | Fp. 46-48.5°C |
| II-56 | 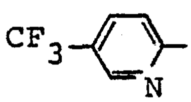 | H | H | 1 | 4-$OCH_3$ | $n_D^{20}$ 1,5335 |

Herstellung von Ausgangsprodukten der allgemeinen Formel (IV):

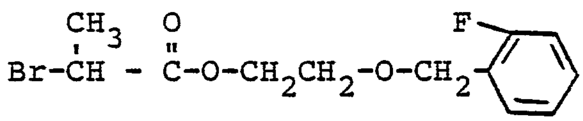

(IV-1)

Eine Lösung von 17,5 g 2-(2-Fluorbenzyloxy)-ethanol und 10,6 g Triethylamin in 150 ml Toluol wurde auf -5°C gekühlt. Unter Rühren wurde bei -5° bis 0°C eine Lösung von 21,6 g 2-Brompropionylbromid in 30 ml Toluol zugetropft. Das Reaktionsgemisch wurde weitere 2 Stunden bei Raumtemperatur gerührt und anschließend nacheinander mit 1 %iger Natronlauge und Wasser gewaschen. Die Toluol-Lösung wurde dann getrocknet und filtriert. Nach Abdestillieren des Toluols bei vermindertem Druck erhielt man 28,2 g 2-Brompropionsäure-/2-(2-fluorbenzyloxy)7-ethylester (IV-1);

$n_D^{20}$ : 1,5038

Auf analoge Weise wurden die in der folgenden Tabelle 2 aufgeführten Verbindungen der allgemeinen Formel (IV) hergestellt:

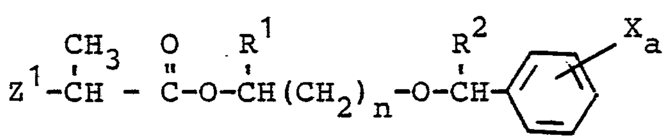

(IV)

Tabelle 2

| Verbindung Nr. | $Z^1$ | $R^1$ | $R^2$ | n | $X_a$ | Physikal.Konstante |
|---|---|---|---|---|---|---|
| IV-2 | Br | H | H | 1 | H | $n_D^{20.5}$ 1.5182 |
| IV-3 | Cl | H | H | 1 | 4-F | $n_D^{20}$ 1.5004 |
| IV-4 | Br | H | H | 1 | 4-F | $n_D^{20}$ 1.5040 |
| IV-5 | Br | H | H | 1 | 2-Cl | $n_D^{20.5}$ 1.5302 |
| IV-6 | Br | H | H | 1 | 4-Cl | $n_D^{20}$ 1.5289 |
| IV-7 | Cl | H | H | 1 | 4-Br | $n_D^{20.5}$ 1.5321 |
| IV-8 | Br | H | H | 1 | 4-Br | $n_D^{20.5}$ 1.5434 |
| IV-9 | Br | H | H | 1 | 2-$CH_3$ | $n_D^{20}$ 1.5186 |
| IV-10 | Br | H | H | 1 | 2-$OCH_3$ | $n_D^{20.5}$ 1.5260 |
| IV-11 | Br | H | H | 1 | 3-$NO_2$ | $n_D^{20}$ 1.5394 |
| IV-12 | Br | H | H | 1 | 2,4-$Cl_2$ | $n_D^{20}$ 1.5395 |
| IV-13 | Br | H | H | 1 | 3,4-$Cl_2$ | $n_D^{20}$ 1.5409 |
| IV-14 | Cl | H | H | 1 | 2,6-$Cl_2$ | $n_D^{20}$ 1.5412 |
| IV-15 | Br | $CH_3$ | H | 1 | H | $n_D^{20.5}$ 1.5100 |

Tabelle 2 (Fortsetzung)

| Verbindung Nr. | $Z^1$ | $R^1$ | $R^2$ | n | $X_a$ | Physikal.Konstante |
|---|---|---|---|---|---|---|
| IV-16 | Br | H | $CH_3$ | 1 | H | $n_D^{20.5}$ 1.5116 |
| IV-17 | Br | H | H | 2 | H | $n_D^{20.5}$ 1.5130 |
| IV-18 | Br | H | H | 1 | 4-$OCH_3$ | $n_D^{20.5}$ 1.5250 |

Beispiel II-31 /nach Verfahren (a)

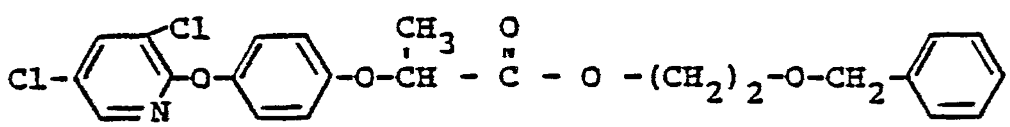

(II-31)

Analog zu Beispiel 1 wurde durch Umsetzung von 4-(3,5-Dichlor-2-pyridyloxy)-phenol-Natriumsalz mit 2-Brompropionsäure-(2-benzyloxy)-ethylester (IV-2) die Verbindung 2-/4-(3,5-Dichlor-2-pyridyloxy)-phenoxy7-propionsäure-(2-benzyloxy)-ethylester (II-31) hergestellt; Schmelzpunkt 59 - 61°C.

Patentansprüche:

1. Selektiv-herbizide Mittel, gekennzeichnet durch einen Gehalt an einer Wirkstoffkombination bestehend aus

(1) N-(2-Benzthiazolyl)-N,N'-dimethylharnstoff (Methabenzthiazuron) der Formel I

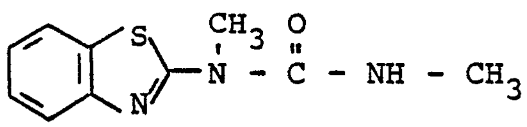

(I)

und

(2) mindestens einem substituierten Phenoxypropionsäureester der allgemeinen Formel II

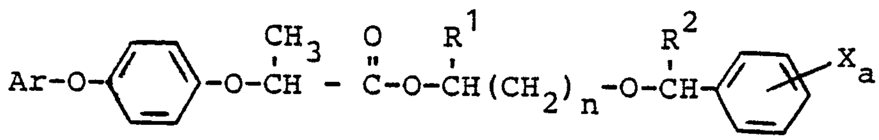

(II)

worin

$R^1$ für Wasserstoff oder Methyl steht,

$R^2$ für Wasserstoff oder Methyl steht,

n für 1 oder 2 steht,

X für Wasserstoff, Methyl, Methoxy, Halogen und/oder Nitro steht,

Ar für den Rest 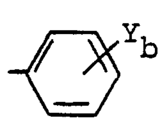 oder 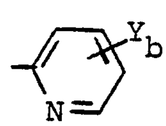

steht,

Y für $C_{1-4}$-Alkyl, Halogen, Trifluormethyl, Nitro und/oder Cyano steht und

b für 1, 2 oder 3 steht.

2. Selektiv-herbizide Mittel gemäß Anspruch 1, dadurch gekennzeichnet, daß in den Wirkstoffkombinationen das Gewichtsverhältnis von Wirkstoff der Formel (I) zu dem Wirkstoff aus der Gruppe (II) zwischen 1:0,001 und 1:20, vorzugsweise zwischen 1:0,01 und 1:10 liegt.

3. Verfahren zur selektiven Bekämpfung von Unkraut in Getreidekulturen, dadurch gekennzeichnet, daß man eine Wirkstoffkombination gemäß Anspruch 1 vor oder nach dem Auflaufen der Pflanzen auf die Getreidefelder einwirken läßt.

4. Verwendung von Wirkstoffkombinationen gemäß Anspruch 1 zur selektiven Bekämpfung von Unkraut in Getreidekulturen.

5. Verfahren zur Herstellung von selektiv-herbiziden Mitteln, dadurch gekennzeichnet, daß man eine Wirkstoffkombination gemäß Anspruch 1 mit Streckmitteln und/oder oberflächenaktiven Mitteln vermischt.

6. Selektiv-herbizide Mittel gemäß Anspruch 1, dadurch gekennzeichnet, daß die Wirkstoffkombination aus Methabenzthiazuron (I) und der Verbindung der Formel II-1

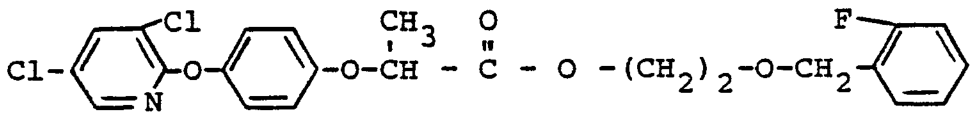

(II-1)

besteht.

7. Selektiv-herbizide Mittel gemäß Anspruch 1, dadurch gekennzeichnet, daß die Wirkstoffkombination aus Methabenzthiazuron (I) und der Verbindung der Formel II-2

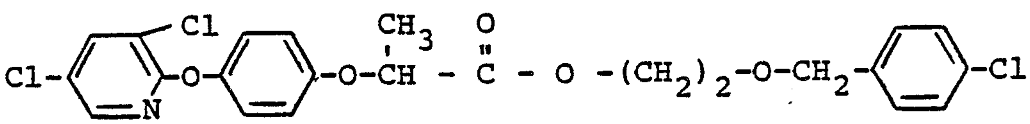

(II-2)

besteht.

8. Selektiv-herbizide Mittel gemäß Anspruch 1, dadurch gekennzeichnet, daß die Wirkstoffkombination aus Methabenzthiazuron (I) und der Verbindung der Formel II-3

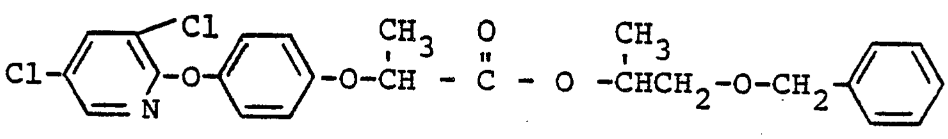

(II-3)

besteht.

9. Selektiv-herbizide Mittel gemäß Anspruch 1, dadurch gekennzeichnet, daß die Wirkstoffkombination aus Methabenzthiazuron (I) und der Verbindung der Formel II-31

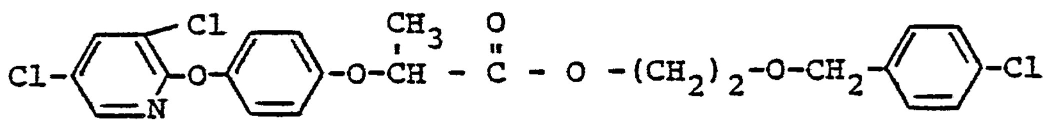

(II-31)

besteht.